# EUROPÄISCHE PATENTSCHRIFT

(11) **EP 1 709 064 B1**
(45) Veröffentlichungstag und Bekanntmachung des Hinweises auf die Patenterteilung: **19.10.2011**
(21) Anmeldenummer: 04803420.1
(22) Anmeldetag: 01.12.2004
(51) Int. Cl.: C07K 14/415, C12N 15/29

(54) **DNA-SEQUENZ UND REKOMBINANTE HERSTELLUNG DES GRASPOLLEN-ALLERGENS LOL P 4**
DNA SEQUENCE, AND RECOMBINANT PRODUCTION OF THE GRASS POLLEN ALLERGEN LOL P 4
SEQUENCE D'ADN ET PRODUCTION PAR RECOMBINAISON DE L'ALLERGENE POLLEN DE PLANTE HERBACEE LOL P 4

(30) Priorität: 16.12.2003 DE 10359352
(43) Veröffentlichungstag der Anmeldung: 11.10.2006
(73) Patentinhaber: Merck Patent GmbH, 64293 Darmstadt (DE)
(72) Erfinder: FIEBIG, Helmut, 21493 Schwarzenbek (DE); NANDY, Andreas, 22175 Hamburg (DE); CROMWELL, Oliver, 23701 Suesel-Fassendorf (DE)
(86) Internationale Anmeldenummer: PCT/EP2004/013663
(87) Internationale Veröffentlichungsnummer: WO 2005/058936

(56) Entgegenhaltungen:
- WO-A-2004/000881
- EKRAMODDOULLAH A K M ET AL: "IMMUNOCHEMICAL CHARACTERIZATION OF A HIGH MOLECULAR WEIGHT BASIC ALLERGEN OF RYE GRASS LOLIUM-PERENNE POLLEN" MOLECULAR IMMUNOLOGY, Bd. 20, Nr. 4, 1983, Seiten 465-474, XP002323104 ISSN: 0161-5890
- JAGGI K S ET AL: "IDENTIFICATION OF TWO DISTINCT ALLERGENIC SITES ON RYEGRASS POLLEN ALLERGEN LOL P IV" JOURNAL OF ALLERGY AND CLINICAL IMMUNOLOGY, Bd. 83, Nr. 4, 1989, Seiten 845-852, XP009045856 ISSN: 0091-6749 in der Anmeldung erwähnt
- JAGGI K S ET AL: "ALLERGENIC FRAGMENTS OF RYEGRASS LOLIUM-PERENNE POLLEN ALLERGEN LOL P-IV" INTERNATIONAL ARCHIVES OF ALLERGY AND APPLIED IMMUNOLOGY, Bd. 89, Nr. 4, 1989, Seiten 342-348, XP009045855 ISSN: 0020-5915 in der Anmeldung erwähnt
- FAHLBUSCH B ET AL: "Detection and quantification of group 4 allergens in grass pollen extracts using monoclonal antibodies" CLINICAL AND EXPERIMENTAL ALLERGY, BLACKWELL SCIENTIFIC PUBLICATIONS, LONDON, GB, Bd. 28, Nr. 7, Juli 1998 (1998-07), Seiten 799-807, XP002260345 ISSN: 0954-7894 in der Anmeldung erwähnt
- DATABASE EMBL 19. November 2004 (2004-11-19), "Lolium perenne partial lolp4 gene for pollen allergen Lol p 4." XP002323105 gefunden im EBI Database accession no. AJ862835

### Sequenz-Protokoll

<110> Merck Patent GmbH
<120> DNA-Sequenz und rekombinante Herstellung des Graspollen-Allergens Lol p 4
<130> P 03/240
<140> DE 10359352.7
   <141> 2003-12-16
<160> 27
<170> PatentIn version 3.1
<210> 1
   <211> 1272
   <212> DNA
   <213> Lol p 4
<220>
   <221> CDS
   <222> (2) .. (1270)
   <223>
<400> 1
   t gcc gtg gtg tgc ggc cgc cgt tac gac gtc cgc atc cgc gta cgc agc      49
     Ala Val Val Cys Gly Arg Arg Tyr Asp Val Arg Ile Arg Val Arg Ser
     1               5                   10                  15
   ggc ggg cac gac tac gag ggc ctc tcg tac cgc tcc ctg cag ccc gag       97
   Gly Gly His Asp Tyr Glu Gly Leu Ser Tyr Arg Ser Leu Gln Pro Glu
               20                  25                  30
   aac ttc gca gtc gtc gac ctc aac cag atg cgg gcg gtg ttg gtg gac      145
   Asn Phe Ala Val Val Asp Leu Asn Gln Met Arg Ala Val Leu Val Asp
           35                  40                  45
   ggt aag gcc cgc acg gcg tgg gtc gac tcc ggc gcg cag ctc ggc gag      193
   Gly Lys Ala Arg Thr Ala Trp Val Asp Ser Gly Ala Gln Leu Gly Glu
       50                  55                  60
   ctc tac tac gcc atc tcc aag tat agc cgc acg ctg gcc ttc ccg gca      241
   Leu Tyr Tyr Ala Ile Ser Lys Tyr Ser Arg Thr Leu Ala Phe Pro Ala
   65                  70                  75                  80
   ggc gtt tgc ccg acc atc ggc gtg ggc ggc aac ctc gcg ggc ggc ggc      289
   Gly Val Cys Pro Thr Ile Gly Val Gly Gly Asn Leu Ala Gly Gly Gly
                   85                  90                  95
   ttc ggt atg ctg ctg cgc aag tac ggc atc gcc gca gag aac gtc atc      337
   Phe Gly Met Leu Leu Arg Lys Tyr Gly Ile Ala Ala Glu Asn Val Ile
               100                 105                 110
   gac gtg aag ctc gtc gac gcc aac ggc aag ctg cac gac aag aag tcc      385
   Asp Val Lys Leu Val Asp Ala Asn Gly Lys Leu His Asp Lys Lys Ser
           115                 120                 125
   atg ggc gac gac cat ttc tgg gcc gtg agg ggt ggc ggc ggc gag agc      433
   Met Gly Asp Asp His Phe Trp Ala Val Arg Gly Gly Gly Gly Glu Ser
       130                 135                 140
   ttc ggc atc gtg gtc tcg tgg cag gtg aag ctc ctg ccg gtg cct ccc      481
   Phe Gly Ile Val Val Ser Trp Gln Val Lys Leu Leu Pro Val Pro Pro
   145                 150                 155                 160
   acg gtg acc atc ttc aag atc ccc aag tca gtc agc gag ggc gcc gtg      529
   Thr Val Thr Ile Phe Lys Ile Pro Lys Ser Val Ser Glu Gly Ala Val
                   165                 170                 175
   gac atc atc aac aag tgg caa ctg gtc gcg cct caa ctt ccc gcc gac      577
   Asp Ile Ile Asn Lys Trp Gln Leu Val Ala Pro Gln Leu Pro Ala Asp
               180                 185                 190
   ctc atg atc cgc atc att gcg atg ggg ccc aag gcc acg ttc gag gcc      625
   Leu Met Ile Arg Ile Ile Ala Met Gly Pro Lys Ala Thr Phe Glu Ala
           195                 200                 205
   atg tac ctc ggc acc tgc aaa acc ctg acg ccg atg atg cag agc aag      673
   Met Tyr Leu Gly Thr Cys Lys Thr Leu Thr Pro Met Met Gln Ser Lys
       210                 215                 220
   ttc ccc gag ctt ggc atg aac gcc tcg cac tgc aac gag atg tca tgg      721
   Phe Pro Glu Leu Gly Met Asn Ala Ser His Cys Asn Glu Met Ser Trp
   225                 230                 235                 240
   atc gag tcc atc ccc ttc gtc cac ctc ggc cat agg gat tcc ctg gag      769
   Ile Glu Ser Ile Pro Phe Val His Leu Gly His Arg Asp Ser Leu Glu
                   245                 250                 255
   ggc gac ctc ctc aac cgg aac aac acc ttc aag ccc ttt gcg gag tac      817
   Gly Asp Leu Leu Asn Arg Asn Asn Thr Phe Lys Pro Phe Ala Glu Tyr
               260                 265                 270
   aaa tcg gac tac gtc tac gag cca ttc ccc aag agc gtg tgg gag cag      865
   Lys Ser Asp Tyr Val Tyr Glu Pro Phe Pro Lys Ser Val Trp Glu Gln
           275                 280                 285
   atc ttc ggc acc tgg ctc gtg aag cct ggt gcg ggg att atg atc ttt        913
   Ile Phe Gly Thr Trp Leu Val Lys Pro Gly Ala Gly Ile Met Ile Phe
       290                 295                 300
   gac ccc tac ggt gcc acc atc agc gct acc cca gaa gcg gcg acg ccg        961
   Asp Pro Tyr Gly Ala Thr Ile Ser Ala Thr Pro Glu Ala Ala Thr Pro
   305                 310                 315                 320
   ttc cct cac cgc aag gga gtc ctc ttc aac atc cag tac gtc aac tac       1009
   Phe Pro His Arg Lys Gly Val Leu Phe Asn Ile Gln Tyr Val Asn Tyr
                   325                 330                 335
   tgg ttc gct ccg gga gcc ggc gcc gcg ccc ttg tca tgg agc aag gaa       1057
   Trp Phe Ala Pro Gly Ala Gly Ala Ala Pro Leu Ser Trp Ser Lys Glu
               340                 345                 350
   atc tac aac tac atg gag ccg tac gtg agc aag aac ccc agg cag gcc       1105
   Ile Tyr Asn Tyr Met Glu Pro Tyr Val Ser Lys Asn Pro Arg Gln Ala
           355                 360                 365
   tac gcc aac tac agg gac atc gac ctc ggg agg aac gag gtg gtg aat       1153
   Tyr Ala Asn Tyr Arg Asp Ile Asp Leu Gly Arg Asn Glu Val Val Asn
       370                 375                 380
   ggc gtc tcc acc tac agc agt ggt aag gtc tgg gga cag aaa tat ttc       1201
   Gly Val Ser Thr Tyr Ser Ser Gly Lys Val Trp Gly Gln Lys Tyr Phe
   385                 390                 395                 400
   aag ggt aac ttc gag agg ctc gcc att acc aag ggc aag gtg gat cct       1249
   Lys Gly Asn Phe Glu Arg Leu Ala Ile Thr Lys Gly Lys Val Asp Pro
                   405                 410                 415
   acg gat tac ttc agg aac gag ca                                        1272
   Thr Asp Tyr Phe Arg Asn Glu
               420
<210> 2
   <211> 423
   <212> PRT
   <213> Lol p 4
<400> 2
   Ala Val Val Cys Gly Arg Arg Tyr Asp Val Arg Ile Arg Val Arg Ser
   1               5                   10                  15
   Gly Gly His Asp Tyr Glu Gly Leu Ser Tyr Arg Ser Leu Gln Pro Glu
               20                  25                  30
   Asn Phe Ala Val Val Asp Leu Asn Gln Met Arg Ala Val Leu Val Asp
           35                  40                  45
   Gly Lys Ala Arg Thr Ala Trp Val Asp Ser Gly Ala Gln Leu Gly Glu
   50 55 60
   Leu Tyr Tyr Ala Ile Ser Lys Tyr Ser Arg Thr Leu Ala Phe Pro Ala
   65 70 75 80
   Gly Val Cys Pro Thr Ile Gly Val Gly Gly Asn Leu Ala Gly Gly Gly
   85 90 95
   Phe Gly Met Leu Leu Arg Lys Tyr Gly Ile Ala Ala Glu Asn Val Ile
   100 105 110
   Asp Val Lys Leu Val Asp Ala Asn Gly Lys Leu His Asp Lys Lys Ser
   115 120 125
   Met Gly Asp Asp His Phe Trp Ala Val Arg Gly Gly Gly Gly Glu Ser
   130 135 140
   Phe Gly Ile Val Val Ser Trp Gln Val Lys Leu Leu Pro Val Pro Pro
   145 150 155 160
   Thr Val Thr Ile Phe Lys Ile Pro Lys Ser Val Ser Glu Gly Ala Val
   165 170 175
   Asp Ile Ile Asn Lys Trp Gln Leu Val Ala Pro Gln Leu Pro Ala Asp
   180 185 190
   Leu Met Ile Arg Ile Ile Ala Met Gly Pro Lys Ala Thr Phe Glu Ala
   195 200 205
   Met Tyr Leu Gly Thr Cys Lys Thr Leu Thr Pro Met Met Gln Ser Lys
   210 215 220
   Phe Pro Glu Leu Gly Met Asn Ala Ser His Cys Asn Glu Met Ser Trp
   225 230 235 240
   Ile Glu Ser Ile Pro Phe Val His Leu Gly His Arg Asp Ser Leu Glu
   245 250 255
   Gly Asp Leu Leu Asn Arg Asn Asn Thr Phe Lys Pro Phe Ala Glu Tyr
   260 265 270
   Lys Ser Asp Tyr Val Tyr Glu Pro Phe Pro Lys Ser Val Trp Glu Gln
   275 280 285
   Ile Phe Gly Thr Trp Leu Val Lys Pro Gly Ala Gly Ile Met Ile Phe
   290 295 300
   Asp Pro Tyr Gly Ala Thr Ile Ser Ala Thr Pro Glu Ala Ala Thr Pro
   305 310 315 320
   Phe Pro His Arg Lys Gly Val Leu Phe Asn Ile Gln Tyr Val Asn Tyr
   325 330 335
   Trp Phe Ala Pro Gly Ala Gly Ala Ala Pro Leu Ser Trp Ser Lys Glu
   340 345 350
   Ile Tyr Asn Tyr Met Glu Pro Tyr Val Ser Lys Asn Pro Arg Gln Ala
   355 360 365
   Tyr Ala Asn Tyr Arg Asp Ile Asp Leu Gly Arg Asn Glu Val Val Asn
   370 375 380
   Gly Val Ser Thr Tyr Ser Ser Gly Lys Val Trp Gly Gln Lys Tyr Phe
   385 390 395 400
   Lys Gly Asn Phe Glu Arg Leu Ala Ile Thr Lys Gly Lys Val Asp Pro
   405 410 415
   Thr Asp Tyr Phe Arg Asn Glu
   420
<210> 3
   <211> 1503
   <212> DNA
   <213> Lol p 4
<220>
   <221> CDS
   <222> (1)..(1503)
   <223>
<400> 3
   tac ttc ccg ccg ccg gct gct aaa gaa gac ttc ctg ggt tgc ctg gtt 48
   Tyr Phe Pro Pro Pro Ala Ala Lys Glu Asp Phe Leu Gly Cys Leu Val
   1 5 10 15
   aaa gaa atc ccg ccg cgt ctg ttg tac gcg aaa tcg tcg ccg gcg tat 96
   Lys Glu Ile Pro Pro Arg Leu Leu Tyr Ala Lys Ser Ser Pro Ala Tyr
   20 25 30
   ccc tca gtc ctg ggg cag acc atc cgg aac tcg agg tgg tcg tcg ccg        144
   Pro Ser Val Leu Gly Gln Thr Ile Arg Asn Ser Arg Trp Ser Ser Pro
           35                  40                  45
   gac aac gtg aag ccg ctc tac atc atc acc ccc acc aac gtc tcc cac        192
   Asp Asn Val Lys Pro Leu Tyr Ile Ile Thr Pro Thr Asn Val Ser His
       50                  55                  60
   atc cag tct gcc gtg gtg tgc ggc cgc cgt tac gac gtc cgc atc cgc        240
   Ile Gln Ser Ala Val Val Cys Gly Arg Arg Tyr Asp Val Arg Ile Arg
   65                  70                  75                  80
   gta cgc agc ggc ggg cac gac tac gag ggc ctc tcg tac cgc tcc ctg        288
   Val Arg Ser Gly Gly His Asp Tyr Glu Gly Leu Ser Tyr Arg Ser Leu
                   85                  90                  95
   cag ccc gag aac ttc gca gtc gtc gac ctc aac cag atg cgg gcg gtg        336
   Gln Pro Glu Asn Phe Ala Val Val Asp Leu Asn Gln Met Arg Ala Val
               100                 105                 110
   ttg gtg gac ggt aag gcc cgc acg gcg tgg gtc gac tcc ggc gcg cag        384
   Leu Val Asp Gly Lys Ala Arg Thr Ala Trp Val Asp Ser Gly Ala Gln
           115                 120                 125
   ctc ggc gag ctc tac tac gcc atc tcc aag tat agc cgc acg ctg gcc        432
   Leu Gly Glu Leu Tyr Tyr Ala Ile Ser Lys Tyr Ser Arg Thr Leu Ala
       130                 135                 140
   ttc ccg gca ggc gtt tgc ccg acc atc ggc gtg ggc ggc aac ctc gcg        480
   Phe Pro Ala Gly Val Cys Pro Thr Ile Gly Val Gly Gly Asn Leu Ala
   145                 150                 155                 160
   ggc ggc ggc ttc ggt atg ctg ctg cgc aag tac ggc atc gcc gca gag        528
   Gly Gly Gly Phe Gly Met Leu Leu Arg Lys Tyr Gly Ile Ala Ala Glu
                   165                 170                 175
   aac gtc atc gac gtg aag ctc gtc gac gcc aac ggc aag ctg cac gac        576
   Asn Val Ile Asp Val Lys Leu Val Asp Ala Asn Gly Lys Leu His Asp
               180                 185                 190
   aag aag tcc atg ggc gac gac cat ttc tgg gcc gtg agg ggt ggc ggc        624
   Lys Lys Ser Met Gly Asp Asp His Phe Trp Ala Val Arg Gly Gly Gly
           195                 200                 205
   ggc gag agc ttc ggc atc gtg gtc tcg tgg cag gtg aag ctc ctg ccg        672
   Gly Glu Ser Phe Gly Ile Val Val Ser Trp Gln Val Lys Leu Leu Pro
       210                 215                 220
   gtg cct ccc acg gtg acc atc ttc aag atc ccc aag tca gtc agc gag        720
   Val Pro Pro Thr Val Thr Ile Phe Lys Ile Pro Lys Ser Val Ser Glu
   225                 230                 235                 240
   ggc gcc gtg gac atc atc aac aag tgg caa ctg gtc gcg cct caa ctt        768
   Gly Ala Val Asp Ile Ile Asn Lys Trp Gln Leu Val Ala Pro Gln Leu
                   245                 250                 255
   ccc gcc gac ctc atg atc cgc atc att gcg atg ggg ccc aag gcc acg        816
   Pro Ala Asp Leu Met Ile Arg Ile Ile Ala Met Gly Pro Lys Ala Thr
               260                 265                 270
   ttc gag gcc atg tac ctc ggc acc tgc aaa acc ctg acg ccg atg atg        864
   Phe Glu Ala Met Tyr Leu Gly Thr Cys Lys Thr Leu Thr Pro Met Met
           275                 280                 285
   cag agc aag ttc ccc gag ctt ggc atg aac gcc tcg cac tgc aac gag      912
   Gln Ser Lys Phe Pro Glu Leu Gly Met Asn Ala Ser His Cys Asn Glu
       290                 295                 300
   atg tca tgg atc gag tcc atc ccc ttc gtc cac ctc ggc cat agg gat      960
   Met Ser Trp Ile Glu Ser Ile Pro Phe Val His Leu Gly His Arg Asp
   305                 310                 315                 320
   tcc ctg gag ggc gac ctc ctc aac cgg aac aac acc ttc aag ccc ttt     1008
   Ser Leu Glu Gly Asp Leu Leu Asn Arg Asn Asn Thr Phe Lys Pro Phe
                   325                 330                 335
   gcg gag tac aaa tcg gac tac gtc tac gag cca ttc ccc aag agc gtg     1056
   Ala Glu Tyr Lys Ser Asp Tyr Val Tyr Glu Pro Phe Pro Lys Ser Val
               340                 345                 350
   tgg gag cag atc ttc ggc acc tgg ctc gtg aag cct ggt gcg ggg att     1104
   Trp Glu Gln Ile Phe Gly Thr Trp Leu Val Lys Pro Gly Ala Gly Ile
           355                 360                 365
   atg atc ttt gac ccc tac ggt gcc acc atc agc gct acc cca gaa gcg     1152
   Met Ile Phe Asp Pro Tyr Gly Ala Thr Ile Ser Ala Thr Pro Glu Ala
       370                 375                 380
   gcg acg ccg ttc cct cac cgc aag gga gtc ctc ttc aac atc cag tac     1200
   Ala Thr Pro Phe Pro His Arg Lys Gly Val Leu Phe Asn Ile Gln Tyr
   385                 390                 395                 400
   gtc aac tac tgg ttc gct ccg gga gcc ggc gcc gcg ccc ttg tca tgg     1248
   Val Asn Tyr Trp Phe Ala Pro Gly Ala Gly Ala Ala Pro Leu Ser Trp
                   405                 410                 415
   agc aag gaa atc tac aac tac atg gag ccg tac gtg agc aag aac ccc     1296
   Ser Lys Glu Ile Tyr Asn Tyr Met Glu Pro Tyr Val Ser Lys Asn Pro
               420                 425                 430
   agg cag gcc tac gcc aac tac agg gac atc gac ctc ggg agg aac gag     1344
   Arg Gln Ala Tyr Ala Asn Tyr Arg Asp Ile Asp Leu Gly Arg Asn Glu
           435                 440                 445
   gtg gtg aat ggc gtc tcc acc tac agc agt ggt aag gtc tgg gga cag     1392
   Val Val Asn Gly Val Ser Thr Tyr Ser Ser Gly Lys Val Trp Gly Gln
       450                 455                 460
   aaa tat ttc aag ggt aac ttc gag agg ctc gcc att acc aag ggc aag     1440
   Lys Tyr Phe Lys Gly Asn Phe Glu Arg Leu Ala Ile Thr Lys Gly Lys
   465                 470                 475                 480
   gtg gat cct acg gat tac ttc agg aac gag cag agc atc ccg ccg ctc     1488
   Val Asp Pro Thr Asp Tyr Phe Arg Asn Glu Gln Ser Ile Pro Pro Leu
                   485                 490                 495
   atc aaa aag tac tga                                                 1503
   Ile Lys Lys Tyr
               500
<210> 4
   <211> 500
   <212> PRT
   <213> Lol p 4
<400> 4
   Tyr Phe Pro Pro Pro Ala Ala Lys Glu Asp Phe Leu Gly Cys Leu Val
   1               5                   10                  15
   Lys Glu Ile Pro Pro Arg Leu Leu Tyr Ala Lys Ser Ser Pro Ala Tyr
               20                  25                  30
   Pro Ser Val Leu Gly Gln Thr Ile Arg Asn Ser Arg Trp Ser Ser Pro
           35                  40                  45
   Asp Asn Val Lys Pro Leu Tyr Ile Ile Thr Pro Thr Asn Val Ser His
       50                  55                  60
   Ile Gln Ser Ala Val Val Cys Gly Arg Arg Tyr Asp Val Arg Ile Arg
   65                  70                  75                  80
   Val Arg Ser Gly Gly His Asp Tyr Glu Gly Leu Ser Tyr Arg Ser Leu
                   85                  90                  95
   Gln Pro Glu Asn Phe Ala Val Val Asp Leu Asn Gln Met Arg Ala Val
               100                 105                 110
   Leu Val Asp Gly Lys Ala Arg Thr Ala Trp Val Asp Ser Gly Ala Gln
           115                 120                 125
   Leu Gly Glu Leu Tyr Tyr Ala Ile Ser Lys Tyr Ser Arg Thr Leu Ala
       130                 135                 140
   Phe Pro Ala Gly Val Cys Pro Thr Ile Gly Val Gly Gly Asn Leu Ala
   145                 150                 155                 160
   Gly Gly Gly Phe Gly Met Leu Leu Arg Lys Tyr Gly Ile Ala Ala Glu
                   165                 170                 175
   Asn Val Ile Asp Val Lys Leu Val Asp Ala Asn Gly Lys Leu His Asp
               180                 185                 190
   Lys Lys Ser Met Gly Asp Asp His Phe Trp Ala Val Arg Gly Gly Gly
           195                 200                 205
   Gly Glu Ser Phe Gly Ile Val Val Ser Trp Gln Val Lys Leu Leu Pro
       210                 215                 220
   Val Pro Pro Thr Val Thr Ile Phe Lys Ile Pro Lys Ser Val Ser Glu
   225                 230                 235                 240
   Gly Ala Val Asp Ile Ile Asn Lys Trp Gln Leu Val Ala Pro Gln Leu
                   245                 250                 255
   Pro Ala Asp Leu Met Ile Arg Ile Ile Ala Met Gly Pro Lys Ala Thr
               260                 265                 270
   Phe Glu Ala Met Tyr Leu Gly Thr Cys Lys Thr Leu Thr Pro Met Met
           275                 280                 285
   Gln Ser Lys Phe Pro Glu Leu Gly Met Asn Ala Ser His Cys Asn Glu
       290                 295                 300
   Met Ser Trp Ile Glu Ser Ile Pro Phe Val His Leu Gly His Arg Asp
   305                 310                 315                 320
   Ser Leu Glu Gly Asp Leu Leu Asn Arg Asn Asn Thr Phe Lys Pro Phe
                   325                 330                 335
   Ala Glu Tyr Lys Ser Asp Tyr Val Tyr Glu Pro Phe Pro Lys Ser Val
               340                 345                 350
   Trp Glu Gln Ile Phe Gly Thr Trp Leu Val Lys Pro Gly Ala Gly Ile
           355                 360                 365
   Met Ile Phe Asp Pro Tyr Gly Ala Thr Ile Ser Ala Thr Pro Glu Ala
       370                 375                 380
   Ala Thr Pro Phe Pro His Arg Lys Gly Val Leu Phe Asn Ile Gln Tyr
   385                 390                 395                 400
   Val Asn Tyr Trp Phe Ala Pro Gly Ala Gly Ala Ala Pro Leu Ser Trp
                   405                 410                 415
   Ser Lys Glu Ile Tyr Asn Tyr Met Glu Pro Tyr Val Ser Lys Asn Pro
               420                 425                 430
   Arg Gln Ala Tyr Ala Asn Tyr Arg Asp Ile Asp Leu Gly Arg Asn Glu
           435                 440                 445
   Val Val Asn Gly Val Ser Thr Tyr Ser Ser Gly Lys Val Trp Gly Gln
       450                 455                 460
   Lys Tyr Phe Lys Gly Asn Phe Glu Arg Leu Ala Ile Thr Lys Gly Lys
   465                 470                 475                 480
   Val Asp Pro Thr Asp Tyr Phe Arg Asn Glu Gln Ser Ile Pro Pro Leu
                   485                 490                 495
   Ile Lys Lys Tyr
               500
<210> 5
   <211> 1503
   <212> DNA
   <213> Phl p 4
<220>
   <221> CDS
   <222> (1).. (1503)
   <223>
<400> 5
   tac ttc ccg ccg ccg gct gct aaa gaa gac ttc ctg ggt tgc ctg gtt       48
   Tyr Phe Pro Pro Pro Ala Ala Lys Glu Asp Phe Leu Gly Cys Leu Val
   1               5                   10                  15
   aaa gaa atc ccg ccg cgt ctg ttg tac gcg aaa tcg tcg ccg gcg tat       96
   Lys Glu Ile Pro Pro Arg Leu Leu Tyr Ala Lys Ser Ser Pro Ala Tyr
               20                  25                  30
   ccc tca gtc ctg ggg cag acc atc cgg aac tcg agg tgg tcg tcg ccg      144
   Pro Ser Val Leu Gly Gln Thr Ile Arg Asn Ser Arg Trp Ser Ser Pro
           35                  40                  45
   gac aac gtg aag ccg ctc tac atc atc acc ccc acc aac gtc tcc cac      192
   Asp Asn Val Lys Pro Leu Tyr Ile Ile Thr Pro Thr Asn Val Ser His
       50                  55                  60
   atc cag tcc gcc gtg gtg tgc ggc cgc cgc cac agc gtc cgc atc cgc      240
   Ile Gln Ser Ala Val Val Cys Gly Arg Arg His Ser Val Arg Ile Arg
   65                  70                  75                  80
   gtg cgc agc ggc ggg cac gac tac gag ggc ctc tcg tac cgg tct ttg      288
   Val Arg Ser Gly Gly His Asp Tyr Glu Gly Leu Ser Tyr Arg Ser Leu
                   85                  90                  95
   cag ccc gag acg ttc gcc gtc gtc gac ctc aac aag atg cgg gcg gtg      336
   Gln Pro Glu Thr Phe Ala Val Val Asp Leu Asn Lys Met Arg Ala Val
               100                 105                 110
   tgg gtg gac ggc aag gcc cgc acg gcg tgg gtg gac tcc ggc gcg cag       384
   Trp Val Asp Gly Lys Ala Arg Thr Ala Trp Val Asp Ser Gly Ala Gln
           115                     120                     125
   ctc ggc gag ctc tac tac gcc atc tat aag gcg agc ccc acg ctg gcg       432
   Leu Gly Glu Leu Tyr Tyr Ala Ile Tyr Lys Ala Ser Pro Thr Leu Ala
       130                     135                     140
   ttc ccg gcc ggc gtg tgc ccg acg atc gga gtg ggc ggc aac ttc gcg       480
   Phe Pro Ala Gly Val Cys Pro Thr Ile Gly Val Gly Gly Asn Phe Ala
   145                     150                     155                     160
   ggc ggc ggc ttc ggc atg ctg ctg cgc aag tac ggc atc gcc gcg gag       528
   Gly Gly Gly Phe Gly Met Leu Leu Arg Lys Tyr Gly Ile Ala Ala Glu
                   165                     170                     175
   aac gtc atc gac gtg aag ctc gtc gac gcc aac ggc aag ctg cac gac       576
   Asn Val Ile Asp Val Lys Leu Val Asp Ala Asn Gly Lys Leu His Asp
               180                     185                     190
   aag aag tcc atg ggc gac gac cat ttc tgg gcc gtc agg ggc ggc ggg       624
   Lys Lys Ser Met Gly Asp Asp His Phe Trp Ala Val Arg Gly Gly Gly
           195                     200                     205
   ggc gag agc ttc ggc atc gtg gtc gcg tgg cag gtg aag ctc ctg ccg       672
   Gly Glu Ser Phe Gly Ile Val Val Ala Trp Gln Val Lys Leu Leu Pro
       210                     215                     220
   gtg ccg ccc acc gtg aca ata ttc aag atc tcc aag aca gtg agc gag       720
   Val Pro Pro Thr Val Thr Ile Phe Lys Ile Ser Lys Thr Val Ser Glu
   225                     230                     235                     240
   ggc gcc gtg gac atc atc aac aag tgg caa gtg gtc gcg ccg cag ctt       768
   Gly Ala Val Asp Ile Ile Asn Lys Trp Gln Val Val Ala Pro Gln Leu
                   245                     250                     255
   ccc gcc gac ctc atg atc cgc atc atc gcg cag ggg ccc aag gcc acg       816
   Pro Ala Asp Leu Met Ile Arg Ile Ile Ala Gln Gly Pro Lys Ala Thr
               260                     265                     270
   ttc gag gcc atg tac ctc ggc acc tgc aaa acc ctg acg ccg ttg atg       864
   Phe Glu Ala Met Tyr Leu Gly Thr Cys Lys Thr Leu Thr Pro Leu Met
           275                     280                     285
   agc agc aag ttc ccg gag ctc ggc atg aac ccc tcc cac tgc aac gag       912
   Ser Ser Lys Phe Pro Glu Leu Gly Met Asn Pro Ser His Cys Asn Glu
       290                     295                     300
   atg tca tgg atc cag tcc atc ccc ttc gtc cac ctc ggc cac agg gac       960
   Met Ser Trp Ile Gln Ser Ile Pro Phe Val His Leu Gly His Arg Asp
   305                     310                     315                     320
   gcc ctc gag gac gac ctc ctc aac cgg aac aac tcc ttc aag ccc ttc      1008
   Ala Leu Glu Asp Asp Leu Leu Asn Arg Asn Asn Ser Phe Lys Pro Phe
                   325                     330                     335
   gcc gaa tac aag tcc gac tac gtc tac cag ccc ttc ccc aag acc gtc      1056
   Ala Glu Tyr Lys Ser Asp Tyr Val Tyr Gln Pro Phe Pro Lys Thr Val
               340                     345                     350
   tgg gag cag atc ctc aac acc tgg ctc gtc aag ccc ggc gcc ggg atc      1104
   Trp Glu Gln Ile Leu Asn Thr Trp Leu Val Lys Pro Gly Ala Gly Ile
           355                 360                 365
   atg atc ttc gac ccc tac ggc gcc acc atc agc gcc acc ccg gag tcc      1152
   Met Ile Phe Asp Pro Tyr Gly Ala Thr Ile Ser Ala Thr Pro Glu Ser
       370                 375                 380
   gcc acg ccc ttc cct cac cgc aag ggc gtc ctc ttc aac atc cag tac      1200
   Ala Thr Pro Phe Pro His Arg Lys Gly Val Leu Phe Asn Ile Gln Tyr
   385                 390                 395                 400
   gtc aac tac tgg ttc gcc ccg gga gcc gcc gcc gcg ccc ctc tcg tgg      1248
   Val Asn Tyr Trp Phe Ala Pro Gly Ala Ala Ala Ala Pro Leu Ser Trp
                   405                 410                 415
   agc aag gac atc tac aac tac atg gag ccc tac gtg agc aag aac ccc      1296
   Ser Lys Asp Ile Tyr Asn Tyr Met Glu Pro Tyr Val Ser Lys Asn Pro
               420                 425                 430
   agg cag gcg tac gca aac tac agg gac atc gac ctc ggc agg aac gag      1344
   Arg Gln Ala Tyr Ala Asn Tyr Arg Asp Ile Asp Leu Gly Arg Asn Glu
           435                 440                 445
   gtg gtc aac gac gtc tcc acc tac gcc agc ggc aag gtc tgg ggc cag      1392
   Val Val Asn Asp Val Ser Thr Tyr Ala Ser Gly Lys Val Trp Gly Gln
       450                 455                 460
   aaa tac ttc aag ggc aac ttc gag agg ctc gcc att acc aag ggc aag      1440
   Lys Tyr Phe Lys Gly Asn Phe Glu Arg Leu Ala Ile Thr Lys Gly Lys
   465                 470                 475                 480
   gtc gat cct acc gac tac ttc agg aac gag cag agc atc ccg ccg ctc      1488
   Val Asp Pro Thr Asp Tyr Phe Arg Asn Glu Gln Ser Ile Pro Pro Leu
                   485                 490                 495
   atc aaa aag tac tga                                                  1503
   Ile Lys Lys Tyr
               500
<210> 6
   <211> 500
   <212> PRT
   <213> Phl p 4
<400> 6
   Tyr Phe Pro Pro Pro Ala Ala Lys Glu Asp Phe Leu Gly Cys Leu Val
   1               5                   10                  15
   Lys Glu Ile Pro Pro Arg Leu Leu Tyr Ala Lys Ser Ser Pro Ala Tyr
               20                  25                  30
   Pro Ser Val Leu Gly Gln Thr Ile Arg Asn Ser Arg Trp Ser Ser Pro
           35                  40                  45
   Asp Asn Val Lys Pro Leu Tyr Ile Ile Thr Pro Thr Asn Val Ser His
       50                  55                  60
   Ile Gln Ser Ala Val Val Cys Gly Arg Arg His Ser Val Arg Ile Arg
   65                  70                  75                  80
   Val Arg Ser Gly Gly His Asp Tyr Glu Gly Leu Ser Tyr Arg Ser Leu
                   85                  90                  95
   Gln Pro Glu Thr Phe Ala Val Val Asp Leu Asn Lys Met Arg Ala Val
               100                 105                 110
   Trp Val Asp Gly Lys Ala Arg Thr Ala Trp Val Asp Ser Gly Ala Gln
           115                 120                 125
   Leu Gly Glu Leu Tyr Tyr Ala Ile Tyr Lys Ala Ser Pro Thr Leu Ala
       130                 135                 140
   Phe Pro Ala Gly Val Cys Pro Thr Ile Gly Val Gly Gly Asn Phe Ala
   145                 150                 155                 160
   Gly Gly Gly Phe Gly Met Leu Leu Arg Lys Tyr Gly Ile Ala Ala Glu
                   165                 170                 175
   Asn Val Ile Asp Val Lys Leu Val Asp Ala Asn Gly Lys Leu His Asp
               180                 185                 190
   Lys Lys Ser Met Gly Asp Asp His Phe Trp Ala Val Arg Gly Gly Gly
           195                 200                 205
   Gly Glu Ser Phe Gly Ile Val Val Ala Trp Gln Val Lys Leu Leu Pro
       210                 215                 220
   Val Pro Pro Thr Val Thr Ile Phe Lys Ile Ser Lys Thr Val Ser Glu
   225                 230                 235                 240
   Gly Ala Val Asp Ile Ile Asn Lys Trp Gln Val Val Ala Pro Gln Leu
                   245                 250                 255
   Pro Ala Asp Leu Met Ile Arg Ile Ile Ala Gln Gly Pro Lys Ala Thr
               260                 265                 270
   Phe Glu Ala Met Tyr Leu Gly Thr Cys Lys Thr Leu Thr Pro Leu Met
           275                 280                 285
   Ser Ser Lys Phe Pro Glu Leu Gly Met Asn Pro Ser His Cys Asn Glu
       290                 295                 300
   Met Ser Trp Ile Gln Ser Ile Pro Phe Val His Leu Gly His Arg Asp
   305                 310                 315                 320
   Ala Leu Glu Asp Asp Leu Leu Asn Arg Asn Asn Ser Phe Lys Pro Phe
                   325                 330                 335
   Ala Glu Tyr Lys Ser Asp Tyr Val Tyr Gln Pro Phe Pro Lys Thr Val
               340                 345                 350
   Trp Glu Gln Ile Leu Asn Thr Trp Leu Val Lys Pro Gly Ala Gly Ile
           355                 360                 365
   Met Ile Phe Asp Pro Tyr Gly Ala Thr Ile Ser Ala Thr Pro Glu Ser
       370                 375                 380
   Ala Thr Pro Phe Pro His Arg Lys Gly Val Leu Phe Asn Ile Gln Tyr
   385                 390                 395                 400
   Val Asn Tyr Trp Phe Ala Pro Gly Ala Ala Ala Ala Pro Leu Ser Trp
                   405                 410                 415
   Ser Lys Asp Ile Tyr Asn Tyr Met Glu Pro Tyr Val Ser Lys Asn Pro
               420                 425                 430
   Arg Gln Ala Tyr Ala Asn Tyr Arg Asp Ile Asp Leu Gly Arg Asn Glu
           435                 440                 445
   Val Val Asn Asp Val Ser Thr Tyr Ala Ser Gly Lys Val Trp Gly Gln
       450                 455                 460
   Lys Tyr Phe Lys Gly Asn Phe Glu Arg Leu Ala Ile Thr Lys Gly Lys
   465                 470                 475                 480
   Val Asp Pro Thr Asp Tyr Phe Arg Asn Glu Gln Ser Ile Pro Pro Leu
                   485                 490                 495
   Ile Lys Lys Tyr
               500
<210> 7
   <211> 12
   <212> PRT
   <213> Dactylus glomerata
<400> 7
   Asp Ile Tyr Asn Tyr Met Glu Pro Tyr Val Ser Lys
   1 5 10
<210> 8
   <211> 11
   <212> PRT
   <213> Dactylus glomerata
<400> 8
   Val Asp Pro Thr Asp Tyr Phe Gly Asn Glu Gln
   1 5 10
<210> 9
   <211> 17
   <212> PRT
   <213> Dactylus glomerata
<400> 9
   Ala Arg Thr Ala Trp Val Asp Ser Gly Ala Gln Leu Gly Glu Leu Ser
   1 5 10 15
   Tyr
<210> 10
   <211> 15
   <212> PRT
   <213> Dactylus glomerata
<400> 10
   Gly Val Leu Phe Asn Ile Gln Tyr Val Asn Tyr Trp Phe Ala Pro
   1 5 10 15
<210> 11
   <211> 11
   <212> PRT
   <213> Cynodon dactylon
<400> 11
   Lys Thr Val Lys Pro Leu Tyr Ile Ile Thr Pro
   1 5 10
<210> 12
   <211> 22
   <212> PRT
   <213> Cynodon dactylon
<400> 12
   Lys Gln Val Glu Arg Asp Phe Leu Thr Ser Leu Thr Lys Asp Ile Pro
   1 5 10 15
   Gln Leu Tyr Leu Lys Ser
   20
<210> 13
   <211> 16
   <212> PRT
   <213> Cynodon dactylon
<400> 13
   Thr Val Lys Pro Leu Tyr Ile Ile Thr Pro Ile Thr Ala Ala Met Ile
   1 5 10 15
<210> 14
   <211> 24
   <212> PRT
   <213> Cynodon dactylon
<400> 14
   Leu Arg Lys Tyr Gly Thr Ala Ala Asp Asn Val Ile Asp Ala Lys Val
   1 5 10 15
   Val Asp Ala Gln Gly Arg Leu Leu
   20
<210> 15
   <211> 14
   <212> PRT
   <213> Cynodon dactylon
<400> 15
   Lys Trp Gln Thr Val Ala Pro Ala Leu Pro Asp Pro Asn Met
   1 5 10
<210> 16
   <211> 15
   <212> PRT
   <213> Cynodon dactylon
<400> 16
   Val Thr Trp Ile Glu Ser Val Pro Tyr Ile Pro Met Gly Asp Lys
   1 5 10 15
<210> 17
   <211> 19
   <212> PRT
   <213> Cynodon dactylon
<220>
   <221> MISC_FEATURE
   <222> (8)..(8)
   <223> undetermined amino acid
<400> 17
   Gly Thr Val Arg Asp Leu Leu Xaa Arg Thr Ser Asn Ile Lys Ala Phe
   1               5                   10                  15
   Gly Lys Tyr
<210> 18
   <211> 23
   <212> PRT
   <213> Cynodon dactylon
<400> 18
   Thr Ser Asn Ile Lys Ala Phe Gly Lys Tyr Lys Ser Asp Tyr Val Leu
   1               5                   10                  15
   Glu Pro Ile Pro Lys Lys Ser
               20
<210> 19
   <211> 13
   <212> PRT
   <213> Cynodon dactylon
<400> 19
   Tyr Arg Asp Leu Asp Leu Gly Val Asn Gln Val Val Gly
   1               5                   10
<210> 20
   <211> 15
   <212> PRT
   <213> Cynodon dactylon
<400> 20
   Ser Ala Thr Pro Pro Thr His Arg Ser Gly Val Leu Phe Asn Ile
   1               5                   10                  15
<210> 21
   <211> 36
   <212> PRT
   <213> Cynodon dactylon
<400> 21
   Ala Ala Ala Ala Leu Pro Thr Gln Val Thr Arg Asp Ile Tyr Ala Phe
   1               5                   10                  15
   Met Thr Pro Tyr Val Ser Lys Asn Pro Arg Gln Ala Tyr Val Asn Tyr
               20                  25                  30
   Arg Asp Leu Asp
           35
<210> 22
   <211> 14
   <212> PRT
   <213> Lolium perenne
<400> 22
   Phe Leu Glu Pro Val Leu Gly Leu Ile Phe Pro Ala Gly Val
   1               5                   10
<210> 23
   <211> 9
   <212> PRT
   <213> Lolium perenne
<400> 23
   Gly Leu Ile Glu Phe Pro Ala Gly Val
   1               5
<210> 24
   <211> 22
   <212> DNA
   <213> Lolium perenne
<400> 24
   ggctcccggg gcgaaccagt ag 22
<210> 25
   <211> 23
   <212> DNA
   <213> Lolium perenne
<400> 25
   accaacgcct cccacatcca gtc 23
<210> 26
   <211> 49
   <212> DNA
   <213> Lolium perenne
<400> 26
   gataagcttg aattctgatt agtacttttt gatcagcggc gggatgctc 49
<210> 27
   <211> 49
   <212> DNA
   <213> Lolium perenne
<400> 27
   gataagcttc tcgagtgatt agtacttttt gatcagcggc gggatgctc 49

## Beschreibung

### Hintergrund der Erfindung

Die vorliegende Erfindung betrifft die Bereitstellung einer DNA-Sequenz des Graspollenhauptallergens Lol p 4. Die Erfindung schließt auch Fragmente und Neukombinationen von Teilsequenzen ein. Die rekombinanten DNA-Moleküle und die abgeleiteten Polypeptide, Fragmente und Neukombinationen von Teilsequenzen können zur Therapie von pollenallergischen Krankheiten genutzt werden. Die rekombinant hergestellten Proteine können zur *In-vitro-* und *In-vivo*-Diagnostik von Pollenallergien eingesetzt werden.

Allergien vom Typ 1 haben weltweite Bedeutung. Bis zu 20 % der Bevölkerung in industrialisierten Ländern leiden unter Beschwerden wie allergischer Rhinitis, Konjunktivitis oder Bronchialasthma. Diese Allergien werden durch in der Luft befindliche Allergene (Aeroallergene), die von Quellen unterschiedlicher Herkunft wie Pflanzenpollen, Milben, Katzen oder Hunden freigesetzt werden, hervorgerufen. Bis zu 40 % dieser Typ 1-Allergiker wiederum zeigen spezifische IgE-Reaktivität mit Gräserpollenallergenen (Freidhoff et al., 1986, J. Allergy Clin. Immunol. 78, 1190-2001).

Bei den Typ 1-Allergie auslösenden Substanzen handelt es sich um Proteine, Glykoproteine oder Polypeptide. Diese Allergene reagieren nach Aufnahme über die Schleimhäute mit den bei sensibilisierten Personen an der Oberfläche von Mastzellen gebundenen IgE-Molekülen. Werden zwei IgE-Moleküle durch ein Allergen miteinander vernetzt, führt dies zur Ausschüttung von Mediatoren (z. B. Histamin, Prostaglandine) und Zytokinen durch die Effektorzelle und damit zu den entsprechenden klinischen Symptomen.

In Abhängigkeit von der relativen Häufigkeit mit der die einzelnen Allergenmoleküle mit den IgE-Antikörpern von Allergikern reagieren, wird zwischen Major- und Minorallergenen unterschieden.

Für das Weidelgras (*Lolium perenne*) wurde das Lol p 1 als ein Hauptallergen identifiziert (Freidhoff et al., 1986, J. Allergy Clin. 78:1190-1201) und seine Primärstruktur aufgeklärt (Perez et al., 1990, J. Biol. Chem. 265:16210-16215). Ein weiteres Hauptallergen ist das LoI p 2 (Freidhoff et al., 1986, J. Allergy Clin. 78:1190-1201) dessen Primärstruktur 1993 beschrieben wurde (Ansari et al., 1989, J. Biol. Chem.: 264:11181-11185). Ein weiteres Hauptallergen des Weidelgrases ist das Lol p 5 (Mattiesen und Löwenstein 1991, Clin. Exp. Allergy 21: 297-307). Die Primärstruktur von Lol p 5 ist ebenfalls bekannt (Ong et al., 1993, Gene 134:235-240). Das Weidelgras enthält weiterhin die Hauptallergene der Gruppen 4 (Fahlbusch et al. 1998, Clin. Exp. Allergy 28: 799-807) und 13 (Petersen et al., 2001, J. Allergy Clin. Imm. 107:856-862).

Das Lol p 4 ist ein typisches basisches Glykoprotein (Jaggi et al, 1989, Int. Arch. Allergy Appl. Immunol. 89:342-348, Jaggi et al., 1989, J. Allergy Clin. Immunol. 83:845-852) und hinsichtlich der Kreuzreaktivität mit spez. IgE-Antikörpern mit dem gut untersuchten Phl p 4, Cyn d 4 und Dac g 4 zu vergleichen (Haavik et al., 1985, Int. Arch. Allergy Appl. Immunol. 78:260-268; Su et al., 1991, Clin. Exp. Allergy 21:449-455; Leduc-Brodard et al., 1996, J. Allergy Clin. Immunol. 98:1065-1072; 14-17).

Diese homologen Moleküle der *Poaceae* bilden die Allergengruppe 4, deren Moleküle eine hohe immunologische Kreuzreaktivität untereinander sowohl mit monoklonalen Mausantikörpern als auch mit humanen IgE-Antikörpern aufweisen (Fahlbusch et al., 1993 Clin. Exp. Allergy 23:51-60; Leduc-Brodard et al., 1996, J. Allergy Clin. Immunol. 98:1065-1072; Su et al., 1996, J. Allergy Clin. Immunol. 97:210; Fahlbusch et al., 1998, Clin. Exp. Allergy 28:799-807; Gavrovic-Jankulovic et al., 2000, Invest. Allergol. Clin. Immunol. 10 (6):361-367; Stumvoll et al. 2002, Biol. Chem. 383:1383-1396; Grote et al., 2002, Biol. Chem. 383:1441-1445; Andersson und Lidholm, 2003, Int. Arch. Allergy Immunol. 130:87-107; Mari, 2003, Clin. Exp. Allergy, 33 (1):43-51).

Im Gegensatz zu den obengenannten Hauptallergenen Lol p 1, Lol p 2, Lol p 5 von *Lolium perenne* ist die Primärstruktur von Lol p 4 noch nicht aufgeklärt.

Von dem Gruppe-4 Allergen aus *Dactylus glomerata* sind bisher lediglich Peptide durch enzymatischen Abbau gewonnen und sequenziert worden:
DIYNYMEPYVSK (SEQ ID NO 7),
VDPTDYFGNEQ (SEQ ID NO 8),
ARTAWVDSGAQLGELSY (SEQ ID NO 9)
und GVLFNIQYVNYWFAP (SEQ ID NO 10, Leduc-Brodard et al., 1996, J. Allergy Clin. Immunol. 98: 1065-1072).

Auch vom Gruppe-4 Allergen des subtropischen Bermuda-Grases (*Cynodon dactylon*) sind durch Proteolyse Peptide erhalten und sequenziert worden:
KTVKPLYIITP (SEQ ID NO 11),
KQVERDFLTSLTKDIPQLYLKS (SEQ ID NO 12),
TVKPLYIITPITAAMI (SEQ ID NO 13),
LRKYGTAADNVIDAKWDAQGRLL (SEQ ID NO 14),
KWQTVAPALPDPNM (SEQ ID NO 15),
VTWIESVPYIPMGDK (SEQ ID NO 16),
GTVRDLLXRTSNIKAFGKY (SEQ ID NO 17),
TSNIKAFGKYKSDYVLEPIPKKS (SEQ ID NO 18),
YRDLDLGVNQWG (SEQ ID NO 19),
SATPPTHRSGVLFNI (SEQ ID NO 20),
und AAAALPTQVTRDIYAFMTPYVSKNPRQAYVNYRDLD (SEQ ID NO 21, Liaw et al., 2001, Biochem. Biophys. Research Communication 280: 738-743).

Für *Lolium perenne* wurden für das basische Gruppe-4 Allergen Peptidfragmente mit den folgenden Sequenzen beschrieben: FLEPVLGLIFPAGV (SEQ ID NO 22) und GLIEFPAGV (SEQ ID NO 23, Jaggi et al., 1989, Int. Arch. Allergy Appl. Immunol. 89: 342-348).

Diese.beschriebenen Peptidsequenzen für *Lolium perenne* und andere Gruppe-4 Allergene haben jedoch bisher nicht zur Aufklärung der Primärstruktur des Lol p 4 Allergens geführt.

Als erste Sequenz eines Allergens der Gruppe 4 wurde von den Erfindern der vorliegenden Patentanmeldung die noch unveröffentlichte Sequenz des PhI p 4 aus *Phleum pratense* aufgeklärt und in der internationalen Anmeldung WO 04/000881 beschrieben.

Die der vorliegenden Erfindung zugrunde liegende Aufgabe bestand daher in der Bereitstellung einer DNA-Sequenz des Lol p 4-Gens, kodierend für ein Allergen mit den immunologischen Eigenschaften des Lol p 4, sowie einer entsprechenden rekombinanten DNA, auf deren Grundlage das Allergen als Protein exprimiert und einer pharmakologisch bedeutsamen Verwertung als solches oder in veränderter Form zugänglich gemacht werden kann. Die Sequenz des Phl p 4 war Ausgangspunkt für die vorliegende Erfindung.

### Verzeichnis der erfindungsgemäßen Sequenzen

- DNA-Sequenz aus dem Lol p 4-Gen (SEQ ID NO 1).
- Aus der DNA-Sequenz gemäß SEQ ID NO 1 abgeleitete Protein-Sequenz (SEQ ID NO 2).
- DNA-Sequenz (SEQ ID NO 3), zusammengesetzt aus den Nukleotiden 1-200 des Phl p 4 (gemäß SEQ ID NO 5), 201-1472 des Lol p 4 (gemäß SEQ ID NO 1) sowie 1473-1503 des Phl p 4 (gemäß SEQ ID NO 5).
- Proteinsequenz (SEQ ID NO 4), zusammengesetzt aus den Aminosäuren 1-67 des Phl p 4 (gemäß SEQ ID NO 6), 68-490 des Lol p 4 (gemäß SEQ ID NO 2) sowie 491-500 des Phl p 4 (gemäß SEQ ID NO 6) mit den Eigenschaften, insbesondere immunologischen Eigenschaften des Lol p 4, kodiert von der DNA-Sequenz gemäß SEQ ID NO 3.
- DNA-Sequenz des Phl p 4 (SEQ ID NO 5), gemäß SEQ ID NO 5 aus der WO 04/000881.
- Proteinsequenz des Phl p 4 (SEQ ID NO 6), gemäß SEQ ID NO 6 aus der WO 04/000881.

### Beschreibung der Erfindung

Mit der vorliegenden Erfindung wird nun erstmals eine DNA-Sequenz des Graspollenhauptallergens Lol p 4 bereit gestellt (SEQ ID NO 1) die für ein Allergen mit den immunologischen Eigenschaften des Lol p 4 kodiert. Gegenstand der vorliegenden Erfindung ist daher ein DNA-Molekül kodierend für ein Allergen mit den Eigenschaften des Lol p 4, entsprechend einer Nukleotidsequenz gemäß SEQ ID NO 1.

Weiterhin ist Gegenstand der Erfindung ein DNA-Molekül kodierend für ein Allergen mit den Eigenschaften des Lol p 4, entsprechend einer Nukleotidsequenz gemäß SEQ ID NO 3, zusammengesetzt aus den Nukleotiden 1-201 des Phl p 4 (gemäß SEQ ID NO 5), 202-1470 des Lol p 4 (SEQ ID NO 1) sowie 1471-1500 des Phl p 4.

Mit der Kenntnis der DNA-Sequenz der natürlich vorkommenden Allergene ist es nun möglich, diese Allergene als rekombinante Proteine herzustellen, die in der Diagnostik und Therapie von allergischen Erkrankungen Verwendung finden können (Scheiner and Kraft, 1995, Allergy 50: 384-391).

Ein klassischer Ansatz zur wirksamen therapeutischen Behandlung von Allergien stellt die Spezifische Immuntherapie oder Hyposensibilisierung dar (Fiebig, 1995, Allergo J. 4 (6): 336-339, Bousquet et al., 1998, J. Allergy Clin. Immunol. 102(4): 558-562). Dabei werden dem Patienten natürliche Allergenextrakte in steigenden Dosen subkutan injiziert. Allerdings besteht bei dieser Methode die Gefahr von allergischen Reaktionen oder sogar eines anaphylaktischen Schocks. Um diese Risiken zu minimieren, werden innovative Präparate in Form von Allergoiden eingesetzt. Dabei handelt es sich um chemisch modifizierte Allergenextrakte, die deutlich reduzierte IgE-Reaktivität, jedoch identische T-Zell-Reaktivität im Vergleich zum nicht behandelten Extrakt aufweisen (Fiebig, 1995, Allergo J. 4 (7): 377-382). Eine noch weitergehende Therapieoptimierung wäre mit rekombinant hergestellten Allergenen möglich. Definierte, ggfs. auf die individuellen Sensibilisierungsmuster der Patienten abgestimmte Cocktails von hochreinen, rekombinant hergestellten Allergenen könnten Extrakte aus natürlichen Allergenquellen ablösen, da diese außer den verschiedenen Allergenen eine größere Zahl von immunogenen, aber nicht allergenen Begleitproteinen enthalten.
Realistische Perspektiven, die zu einer sicheren Hyposensibilisierung mit Expressionsprodukten führen können, bieten gezielt mutierte rekombinante Allergene, bei denen IgE-Epitope spezifisch deletiert werden, ohne die für die Therapie essentiellen T-Zell Epitope zu beeinträchtigen (Schramm et al., 1999, J. Immunol. 162: 2406-2414).

Eine weitere Möglichkeit zur therapeutischen Beeinflussung des gestörten TH-Zell-Gleichgewichtes bei Allergikern ist die immuntherapeutische DNA-Vakzinierung. Dabei handelt es sich um eine Behandlung mit expressionsfähiger DNA, die für die relevanten Allergene kodiert. Erste experimentelle Belege für die allergenspezifische Beeinflussung der Immunantwort konnte an Nagern durch Injektion von Allergen-kodierender DNA erbracht werden (Hsu et al., 1996, Nature Medicine 2 (5): 540-544).

Gegenstand der vorliegenden Erfindung ist daher auch ein vor- oder nachstehend beschriebenes DNA-Molekül bzw. ein entsprechender rekombinanter Expressionsvektor als Arzneimittel.

Die entsprechenden rekombinant hergestellten Proteine können zur Therapie sowie zur *in vitro-* und *in vivo*-Diagnostik von Pollenallergien eingesetzt werden.

Zur Herstellung des rekombinanten Allergens wird die klonierte Nukleinsäure in einen Expressionsvektor ligiert und dieses Konstrukt in einem geeigneten Wirtsorganismus exprimiert. Nach biochemischer Reinigung steht dieses rekombinante Allergen zur Detektion von IgE-Antikörpern in etablierten Verfahren zur Verfügung.

Gegenstand der vorliegenden Erfindung ist daher weiterhin ein rekombinanter Expressionsvektor, enthaltend ein vor- oder nachstehend beschriebenes DNA-Molekül, funktionell verbunden mit einer Expressionskontrollsequenz und ein Wirtsorganismus, transformiert mit besagtem DNA-Molekül oder besagtem Expressionsvektor.

Ebenfalls erfindungsgegenständlich ist die Verwendung mindestens eines zuvor beschriebenen DNA-Moleküls oder mindestens eines zuvor beschriebenen Expressionsvektors zur Herstellung eines Arzneimittels zur immuntherapeutischen DNA-Vakzinierung von Patienten mit Allergien, an deren Auslösung Gruppe-4-Allergene der *Poaceae,* insbesondere Lol p 4, beteiligt sind und/oder zur Prävention solcher Allergien.

Wie bereits ausgeführt kann die Erfindung als eine essentielle Komponente in einem rekombinanten allergen- oder nukleinsäurehaltigen Präparat zur spezifischen Immuntherapie angewendet werden. Hierbei bieten sich mehrere Möglichkeiten. Zum einen kann das in der Primärstruktur unveränderte Protein Bestandteil des Präparates sein. Zum anderen kann durch gezielte Deletion von IgE-Epitopen des Gesamtmoleküls oder der Herstellung von einzelnen Fragmenten, die für T-Zell Epitope kodieren, erfindungsgemäß eine hypoallergene (allergoide) Form zur Therapie verwendet werden, um unerwünschte Nebenwirkungen zu vermeiden. Schließlich wird durch die Nukleinsäure an sich, wenn sie mit einem eukaryontischen Expressionsvektor ligiert wird, ein Präparat geschaffen, das direkt appliziert den allergischen Immunzustand im therapeutischen Sinne verändert.

Desweiteren handelt es sich bei der vorliegenden Erfindung um die von einem oder mehreren der zuvor beschriebenen DNA-Moleküle kodierten Polypeptide, vorzugsweise in ihrer Eigenschaft als Arzneimittel. Es handelt sich bei den Polypeptiden um ein Protein entsprechend einer Aminosäuresequenz gemäß SEQ ID NO 2 bzw. eines Proteins. welches diese Aminosäuresequenz oder einen Teil dieser Sequenz enthält, mit den Eigenschaften, insbesondere immunologischen Eigenschaften des Lol p 4 sowie um ein Protein entsprechend einer Aminosäuresequenz gemäß SEQ ID NO 4 mit den Eigenschaften, insbesondere immunologischen Eigenschaften des Lol p 4.

Die Erfindung betrifft demgemäß auch ein Verfahren zur Herstellung solcher Polypeptide durch Kultivieren eines Wirtsorganismus und Gewinnung des entsprechenden Polypeptides aus der Kultur.

Ebenfalls erfindungsgegenständlich ist die Verwendung mindestens eines zuvor beschriebenen Polypeptides bzw. Proteins zur Herstellung eines Arzneimittels zur Diagnose und/oder Behandlung von Allergien, an deren Auslösung Gruppe-4-Allergene der *Poaceae,* insbesondere Lol p 4, beteiligt sind sowie zur Prävention solcher Allergien.

Bei der Ermittlung der Protein- und DNA-Sequenz des Lol p 4 wurde wie folgt vorgegangen:
Die erfindungsgemäße Lol p 4-DNA-Sequenz gemäß SEQ ID NO 1 wurde durch PCR mit spezifischen Primern (Tab. 1), die von der Phl p 4 Sequenz, germäß SEQ ID NO 5 wie in der WO 04/000881 beschrieben, abgeleitet wurden, amplifiziert, kloniert und sequenziert. Insgesamt wurden 7 Klone analysiert. Die Analyse der Klone ergab eine einheitliche Sequenz. Drei Lol p 4-DNA-Sequenzen wurden durch PCR mit den Primern #87 und #83 erhalten. Die mit diesen Primern amplifizierte Lol p 4-DNA-Sequenz kodiert für die korrespondierenden Aminosäuren 68-401, bezogen auf die Nummerierung des reifen Phl p 4 gemäß SEQ ID NO 6. Zwei weitere Klone wurden durch PCR mit den Primern #87 und #189 erhalten. Die mit diesen Primern amplifizierte Lol p 4-DNA-Sequenz kodiert für die korrespondierenden Aminosäuren 68-490 (Nummerierung entsprechend Phl p 4-Sequenz). Zwei Klone wurden durch PCR mit den Primern #87 und #131 erhalten. Die amplifizierte Lol p 4-DNA-Sequenz kodiert ebenfalls für die korrespondierenden Aminosäuren 68-490 (Nummerierung entsprechend Phl p 4-Sequenz). Die Primer #131 und #189 entsprechen den Codons für die letzten 10 Aminosäuren des Phl p 4 Proteins und überspannen das Stoppcodon.

Die erfindungsgemäße DNA-Sequenz gemäß SEQ ID NO 3 wurde nach an sich bekannten Methoden erhalten (PCR-Technik mit überlappenden Primern).

Zur Herstellung der rekombinanten erfindungsgemäßen Allergene wurden die DNA-Sequenzen gemäß SEQ ID NO 1 bzw. 3 in Expressionsvektoren (z.B. pProEx, pSE 380) eingebaut. Für die aus der Proteinsequenzierung bekannten N-terminalen Aminosäuren wurden *E. coli* optimierte Codons verwendet.

Nach der Transformation in *E. coli*, der Expression und der Reinigung der rekombinanten erfindungsgemäßen Allergene durch verschiedene Trenntechniken wurde die erhaltenen Proteine einem Refoldingprozess unterworfen.

Beide Allergene können zur hochspezifischen Diagnostik von Graspollenallergien eingesetzt werden. Diese Diagnostik kann *in vitro* durch die Detektion von spezifischen Antikörpern (IgE, IgG1 - 4, IgA) und die Reaktion mit IgE-beladenen Effektorzellen (z. B. Basophile aus dem Blut) oder *in vivo* durch Hauttest-Reaktionen und Provokation am Reaktionsorgan erfolgen.

Die Reaktion der erfindungsgemäßen Allergene mit T-Lymphozyten von Graspollenallergikern können durch die allergenspezifische Stimulierung der T-Lymphozyten zur Proliferation und Zytokinsynthese sowohl mit T-Zellen in frisch präparierten Blutlymphozyten als auch an etablierten nLol p 4-reaktiven T-Zell-Linien und -Klonen nachgewiesen werden.

Durch ortsgerichte Mutagenese wurden die für die Cysteine kodierenden Tripletts so verändert, dass sie für andere Aminosäuren, bevorzugt Serin, kodieren. Es wurden sowohl Varianten hergestellt, bei denen einzelne Cysteine ausgetauscht wurden, als auch solche, bei denen verschiedene Kombinationen von 2 Cysteinresten bzw. alle Cysteine verändert wurden. Die exprimierten Proteine dieser Cysteinpunktmutanten weisen eine stark reduzierte bzw. fehlende Reaktivität mit IgE Antikörpern von Allergikern auf, reagieren jedoch mit den T-Lymphozythen dieser Patienten.

Gegenstand der vorliegenden Erfindung ist daher weiterhin ein vor- oder nachstehend beschriebenes DNA-Molekül, bei dem durch ortsgerichtete Mutagenese einer, mehrere oder alle Cystein-Reste des entsprechenden Polypeptids gegen eine andere Aminosäure ausgetauscht wurden.

Die immunmodulatorische Aktivität von hypoallergenen Fragmenten, die Polypeptiden mit T-Zell-Epitopen entsprechen, sowie die der hypoallergenen Cystein-Austausche kann durch ihre Reaktion mit T-Zellen von Graspollenallergikern nachgewiesen werden.

Solche hypoallergenen Fragmente bzw. Punktmutanten der Cysteine können als Präparate zur Hyposensibilisierung von Allergikern eingesetzt werden, da sie mit gleicher Effektivität mit den T-Zellen reagieren, jedoch aufgrund der verminderten oder ganz fehlenden IgE-Reaktivität zu geringeren IgE-vermittelten Nebenwirkungen führen.

Werden die für die erfindungsgemäßen hypoallergenen Allergen-Varianten kodierenden Nukleinsäuren oder die unveränderten erfindungsgemäßen DNA-Moleküle mit einem humanen Expressionsvektor ligiert, können diese Konstrukte ebenfalls als Präparate für eine Immuntherapie (DNA-Vakzinierung) angewendet werden.

Schließlich sind Gegenstand der vorliegenden Erfindung pharmazeutische Zubereitungen, enthaltend mindestens ein zuvor beschriebenes DNA-Molekül oder mindestens einen zuvor beschriebenen Expressionsvektor und gegebenenfalls weitere Wirk- und/oder Hilfsstoffe zur immuntherapeutischen DNA-Vakzinierung von Patienten mit Allergien, an deren Auslösung Gruppe-4-Allergene der *Poaceae*, insbesondere Lol p 4, beteiligt sind und/oder zur Prävention solcher Allergien.

Eine weitere Gruppe von erfindungsgemäßen pharmazeutischen Zubereitungen enthält anstelle der DNA mindestens ein zuvor beschriebenes Polypeptid und eignet sich zur Diagnose und/oder Behandlung besagter Allergien.

Pharmazeutische Zubereitungen im Sinne der vorliegenden Erfindung enthaltend als Wirkstoffe ein erfindungsgemäßes Polypeptid oder einen Expressionsvektor und/oder deren jeweilige pharmazeutisch verwendbaren Derivate, einschließlich deren Mischungen in allen Verhältnissen. Hierbei können die erfindungsgemäßen Wirkstoffe zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.
Als Hilfsstoffe sind immunstimulierende DNA oder Oligonukleotide mit CpG-Motiven besonders geeignet.

Diese Zubereitungen können als Therapeutika oder Diagnostika in der Human- oder Veterinärmedizin verwendet werden. Als Trägerstoffe kommen organische oder anorganische Substanzen in Frage, die sich für die parenterale Applikation eignen und die Wirkung des erfindungsgemäßen Wirkstoffs nicht negativ beeinflussen. Zur parenteralen Anwendung dienen insbesondere Lösungen, vorzugsweise ölige oder wässrige Lösungen, ferner Suspensionen, Emulsionen oder Implantate. Der erfindungsgemäße Wirkstoff kann auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen und/oder mehrere weitere Wirkstoffe enthalten.
Weiterhin können durch entsprechende Formulierung des erfindungsgemäßen Wirkstoffs Depotpräparate - zum Beispiel durch Adsorption an Aluminiumhydroxid - erhalten werden.

Die Erfindung dient somit auch zur Verbesserung der *in vitro* Diagnostik im Rahmen einer Allergen-Komponenten auflösenden Identifizierung des patientenspezifischen Sensibilisierungsspektrums. Die Erfindung dient ebenfalls zur Herstellung von deutlich verbesserten Präparaten zur spezifischen Immuntherapie von Gräserpollenallergien.

**Tabelle 1 Verwendete Primer**

| **Primer nummer** | **SEQ ID NO** | **Sequenz** |
|---|---|---|
| #83 | 24 | GGCTCCCGGGGCGAACCAGTAG |
| #87 | 25 | ACCAACGCCTCCCACATCCAGTC |
| #131 | 26 | |
| #189 | 27 | GATAAGCTTCTCGAGTGATTAGTACIIIIIGATCAGCGGCGGGATGCTC |

## Patentansprüche

1. Ein DNA-Molekül kodierend für ein Allergen mit den Eigenschaften des Lol p 4, entsprechend einer Nukleotidsequenz, ausgewählt aus einer der Sequenzen gemäß SEQ ID NO 1 und 3.

2. Ein DNA-Molekül, entsprechend einer Teilsequenz des DNA-Moleküls gemäß Anspruch 1, welches für ein immunmodulatorisches, T-Zell-reaktives Fragment von Lol p 4 kodiert.

3. Ein DNA-Molekül, entsprechend einer Kombination aus Teilsequenzen des DNA-Moleküls gemäß Anspruch 1, welche für ein immunmodulatorisches, T-Zell-reaktives Fragment von Lol p 4 kodieren.

4. Ein DNA-Molekül, entsprechend einer Nukleotidsequenz gemäß einem oder mehreren der Ansprüche 1 bis 3, **dadurch gekennzeichnet, dass** eine Mutation zum Austausch eines, mehrerer oder aller Cysteine des entsprechenden Polypeptids gegen eine andere Aminosäure führt.

5. Ein rekombinanter DNA-Expressionsvektor oder ein Klonierungssystem, enthaltend ein DNA-Molekül gemäß einem oder mehreren der Ansprüche 1 bis 4, funktionell verbunden mit einer Expressionskontrollsequenz.

6. Ein Wirtsorganismus, transformiert mit einem DNA-Molekül gemäß einem oder mehreren der Ansprüche 1 bis 4 oder einem Expressionsvektor gemäß Anspruch 5.

7. Ein Verfahren zur Herstellung eines Polypeptids, kodiert durch eine DNA-Sequenz gemäß einem oder mehreren der Ansprüche 1 bis 4, durch Kultivieren eines Wirtsorganismus gemäß Anspruch 6 und Gewinnung des entsprechenden Polypeptids aus der Kultur.

8. Ein rekombinantes Polypeptid, welches von einer DNA-Sequenz gemäß einem oder mehreren der Ansprüche 1 bis 4 kodiert wird.

9. Ein Polypeptid gemäß Anspruch 8 als Arzneimittel.

10. Eine pharmazeutische Zubereitung, enthaltend mindestens ein Polypeptid gemäß Anspruch 9 und gegebenenfalls weitere Wirk- und/oder Hilfsstoffe zur Diagnose und/oder Behandlung von Allergien, an deren Auslösung Gruppe-4-Allergene der *Poaceae* beteiligt sind.

11. Verwendung mindestens eines Polypeptids gemäß Anspruch 9 zur Herstellung eines Arzneimittels zur Diagnose und/oder Behandlung von Allergien, an deren Auslösung Gruppe-4-Allergene der *Poaceae* beteiligt sind und/oder zur Prävention solcher Allergien.

12. Ein DNA-Molekül gemäß einem oder mehreren der Ansprüche 1 bis 4 als Arzneimittel.

## Claims

1. A DNA molecule encoding for an allergen having the properties of Lol p 4, corresponding to a nucleotide sequence selected from one of the sequences in accordance with SEQ ID NO 1 and 3.

2. A DNA molecule, corresponding to a partial sequence of the DNA molecule according to Claim 1 which encodes for an immunomodulatory, T-cell-reactive fragment of Lol p 4.

3. A DNA molecule, corresponding to a combination of partial sequences of the DNA molecule according to Claim 1 which encode for an immunomodulatory, T-cell-reactive fragment of Lol p 4.

4. A DNA molecule, corresponding to a nucleotide sequence according to one or more of Claims 1 to 3, **characterised in that** mutation results in the replacement of one, more than one or all cysteines of the corresponding polypeptide with another amino acid.

5. A recombinant DNA expression vector or a cloning system, comprising a DNA molecule according to one or more of Claims 1 to 4, functionally linked to an expression control sequence.

6. A host organism transformed with a DNA molecule according to one or more of Claims 1 to 4 or an expression vector according to Claim 5.

7. A process for the preparation of a polypeptide encoded by a DNA sequence according to one or more of Claims 1 to 4 by cultivation of a host organism according to Claim 6 and isolation of the corresponding polypeptide from the culture.

8. A recombinant polypeptide which is encoded by a DNA sequence according to one or more of Claims 1 to 4.

9. A polypeptide according to Claim 8 as medicament.

10. A pharmaceutical composition comprising at least one polypeptide according to Claim 9 and optionally further active compounds and/or adjuvants for the diagnosis and/or treatment of allergies in the triggering of which group 4 allergens from the *Poaceae* are involved.

11. Use of at least one polypeptide according to Claim 9 for the preparation of a medicament for the diagnosis and/or treatment of allergies in the triggering of which group 4 allergens from the *Poaceae* are involved and/or for the prevention of such allergies.

12. A DNA molecule according to one or more of Claims 1 to 4 as medicament.

## Revendications

1. Molécule d'ADN codant pour un allergène présentant les propriétés de Lol p 4, correspondant à une séquence de nucléotides choisie parmi l'une des séquences conformément à SEQ ID NO 1 et 3.

2. Molécule d'ADN, correspondant à une séquence partielle de la molécule d'ADN selon la revendication 1, laquelle code pour une propriété immunomodulatrice, à savoir fragment réactif de cellule T de Lol p 4.

3. Molécule d'ADN, correspondant à une combinaison de séquences partielles de la molécule d'ADN selon la revendication 1, laquelle code pour une propriété immunomodulatrice, à savoir fragment réactif de cellule T de Lol p 4.

4. Molécule d'ADN, correspondant à une séquence de nucléotides selon une ou plusieurs des revendications 1 à 3, **caractérisée en ce qu'**une mutation aboutit au remplacement d'une cystéine, plus d'une cystéine ou toutes les cystéines du polypeptide correspondant par un autre acide aminé.

5. Vecteur d'expression d'ADN recombinant ou système de clonage, comprenant une molécule d'ADN selon une ou plusieurs des revendications 1 à 4, fonctionnellement lié à une séquence de contrôle d'expression.

6. Organisme hôte transformé avec une molécule d'ADN selon une ou plusieurs des revendications 1 à 4 ou un vecteur d'expression selon la revendication 5.

7. Procédé pour la préparation d'un polypeptide codé par une séquence d'ADN selon une ou plusieurs des revendications 1 à 4 par culture d'un organisme hôte selon la revendication 6 et isolement du polypeptide correspondant vis-à-vis de la culture.

8. Polypeptide recombinant qui est codé par une séquence d'ADN selon une ou plusieurs des revendications 1 à 4.

9. Polypeptide selon la revendication 8 en tant que médicament.

10. Composition pharmaceutique comprenant au moins un polypeptide selon la revendication 9 et, en option, d'autres composés actifs et/ou adjuvants pour le diagnostic et/ou le traitement d'allergies dans le déclenchement desquelles des allergènes du groupe 4 issus de *Poaceae* sont mis en jeu.

11. Utilisation d'au moins un polypeptide selon la revendication 9 pour la préparation d'un médicament pour le diagnostic et/ou le traitement d'allergies dans le déclenchement desquelles des allergènes du groupe 4 issus de *Poaceae* sont mis en jeu et/ou pour la prévention de ces allergies.

12. Molécule d'ADN selon une ou plusieurs des revendications 1 à 4 en tant que médicament.
